# EUROPEAN PATENT APPLICATION

(11) **EP 2 907 814 A1**
(43) Date of publication of application: **19.08.2015**
(21) Application number: 13844913.7
(22) Date of filing: 02.10.2013
(51) Int. Cl.: C07D 405/06

(54) **METHOD FOR PRODUCING CYCLOALKANOL DERIVATIVE, AND METHOD FOR PRODUCING AZOLE DERIVATIVE**

(30) Priority: 11.10.2012 JP 2012226249
(71) Applicant: Kureha Corporation, Tokyo 103-8552 (JP)
(72) Inventor: MASANO Taiga, Tokyo 103-8552 (JP); SUDO Keiichi, Tokyo 103-8552 (JP)
(74) Representative: Zimmermann & Partner Patentanwälte mbB
(86) International application number: PCT/JP2013/076780
(87) International publication number: WO 2014/057844

(57) **Abstract**

Provided are: a method for producing a cycloalkanol derivative that enables the production of an azole derivative at low cost and with high yield; and a method for producing an azole derivative.

In a process of producing a cycloalkanol derivative represented by general formula (I), a reaction step of azolylmethylating a cycloalkanone derivative represented by general formula (II) is carried out using an azole compound represented by general formula (III) in the presence of a sulfur ylide. In this step, the sulfur ylide is produced from a sulfonium compound or sulfoxonium compound and an alkali metal methoxide in a reaction system.

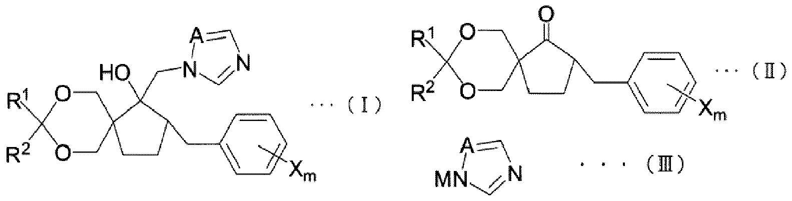

## Description

### TECHNICAL FIELD

The present invention relates to a method for producing a cycloalkanol derivative, and to a method for producing an azole derivative. More specifically, the present invention relates to a method for producing a cycloalkanol derivative, which is useful as an intermediate compound of an azole derivative used as an active ingredient for an agricultural and horticultural agent, also relates to a method for producing an azole derivative by using a cycloalkanol derivative produced using the aforementioned method.

### BACKGROUND ART

Some known azolylmethylcyclopentanol derivatives exhibit excellent activity as active ingredients in agricultural and horticultural disease control agents and agents for protecting industrial materials. For example, Patent Document 1 discloses a 2-(halogenated hydrocarbon-substituted)-5-benzyl-1-azolylmethylcyclopentanol derivative, which exhibits low toxicity to humans and animals and which exhibits a high controlling effect on a wide range of plant diseases and a high growth regulating effect on a variety of agricultural and horticultural plants.

Patent Document 1 discloses a production example in which a solid alkali metal butoxide is used as a base when subjecting an intermediate to azolylmethylation, which is a part of a process for producing 2-(halogenated hydrocarbon-substituted)-5-benzyl-1-azolylmethylcyclopentanol derivative. In addition, Patent Document 2 discloses a production example in which an alkali metal butoxide is used as a base when subjecting an intermediate to azolylmethylation, which is a part of a process for producing an azolylmethylcycloalkanol derivative.

### CITATION LISTS

### Patent Literatures

Patent Document 1: WO/2011/070771
Patent Document 2: Japanese Unexamined Patent Application Publication No. H01-301664A
Patent Document 3: Japanese Unexamined Patent Application Publication No. H01-093574A

### SUMMARY OF INVENTION

### Technical Problem

However, it cannot be said that conventional methods for producing azolylmethylcyclopentanol derivatives (hereinafter referred to as azole derivatives) are sufficiently inexpensive or capable of obtaining azole derivatives with high yields, and further improvements are required.

Therefore, a main objective of the present invention is to provide a method for producing a cycloalkanol derivative that enables the production of an azole derivative at low cost and with high yield; and a method for producing an azole derivative.

### Solution to Problem

As a result of diligent research into how to solve the problems mentioned above, the inventors of the present invention found that there was still room for improvement in a method for producing a cycloalkanol derivative used as an intermediate compound for an azole derivative, and succeeded in completing the present invention.

That is, the method for producing a cycloalkanol derivative according to the present invention is a method for producing a cycloalkanol derivative represented by general formula (I) below, and is characterized by including an azolylmethylation reaction step of azolylmethylating a cycloalkanone derivative represented by general formula (II) below using an azole compound represented by general formula (III) below in the presence of a sulfur ylide, wherein the sulfur ylide is produced from a sulfonium compound or sulfoxonium compound and an alkali metal methoxide in a reaction system in the azolylmethylation reaction step.

[Formula 1]

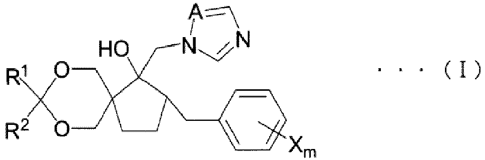

Here, in formula (I) above, R¹ and R² are each independently a hydrogen atom, an alkyl group having from 1 to 4 carbon atoms, a phenyl group or a benzyl group. Moreover, one or more hydrogen atoms on these phenyl groups and benzyl groups may be substituted with alkyl groups having from 1 to 4 carbon atoms, alkoxy groups having from 1 to 4 carbon atoms, or halogen atoms. In addition, A is a nitrogen atom or a methine group. Furthermore, X is a halogen atom, an alkyl group having from 1 to 4 carbon atoms, a haloalkyl group having from 1 to 4 carbon atoms, an alkoxy group having from 1 to 4 carbon atoms, a haloalkoxy group having from 1 to 4 carbon atoms, a phenyl group, a cyano group or a nitro group, and m is an integer from 0 to 5. Moreover, in cases where m is 2 or higher, the plurality of X groups may be different from each other.

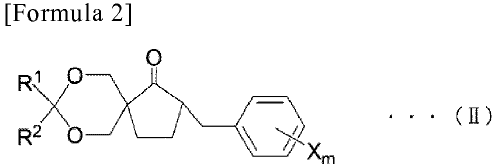

In formula (II) above, R¹, R², X and m are the same as R¹, R², X and m in formula (I) above.

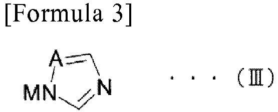

In formula (III) above, M is an alkali metal atom and A is the same as A in formula (I) above.

Moreover, in the present invention, "reaction system" means either a single reaction that occurs in a reaction vessel or a plurality of reactions that occur successively in a reaction vessel. That is, in the present invention, even in the case of a product obtained from a raw material via an intermediate, reactions that occur successively in a reaction vessel are regarded as being the same reaction system.

### Advantageous Effects of Invention

According to the present invention, because an alkali metal methoxide is used when producing the sulfur ylide in the reaction system in the azolylmethylation reaction step in the process for producing the cycloalkanol derivative, it is possible to produce an azole derivative at lower cost and with higher yield compared to conventional production methods.

### Description of Embodiments

A preferred embodiment for carrying out the present invention will now be explained. Moreover, the embodiment explained below illustrates a single representative example of the present invention, and it should not be interpreted that the scope of the present invention is narrowed by this embodiment.

### <Cycloalkanol derivative (I)>

In a method for producing a cycloalkanol derivative according to an embodiment of the present invention, a cycloalkanol derivative represented by general formula (I) below (hereinafter referred to as cycloalkanol derivative (I)) is produced. Before explaining the production method in detail, the structure of cycloalkanol derivative (I) will be explained.

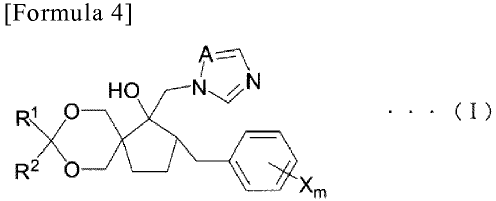

In formula (I) above, R¹ and R² are each independently a hydrogen atom, an alkyl group having from 1 to 4 carbon atoms, a phenyl group or a benzyl group.

Examples of alkyl groups having from 1 to 4 carbon atoms include methyl groups, ethyl groups, n-propyl groups, 1-methylethyl groups, 1-methylpropyl groups, 2-methylpropyl groups, n-butyl groups and 1,1-dimethylethyl groups.

One or more hydrogen atoms in the phenyl groups in R¹ and/or R² and one or more hydrogen atoms in the phenyl moiety of the benzyl groups in R¹ and/or R² may be substituted with alkyl groups having from 1 to 4 carbon atoms, alkoxy groups having from 1 to 4 carbon atoms, or halogen atoms. Examples of substituent alkyl groups having from 1 to 4 carbon atoms include methyl groups, ethyl groups, n-propyl groups, 1-methylethyl groups, 1-methylpropyl groups, 2-methylpropyl groups, n-butyl groups and 1,1-dimethylethyl groups. Examples of substituent alkoxy groups having from 1 to 4 carbon atoms include methoxy groups, ethoxy groups and n-propoxy groups. Examples of substituent halogen atoms include fluorine atoms, chlorine atoms and bromine atoms.

Of these, it is preferable for R¹ and R² to be each independently a hydrogen atom or an alkyl group having from 1 to 4 carbon atoms, more preferably a hydrogen atom or an alkyl group having from 1 to 2 carbon atoms, and further preferably a hydrogen atom or a methyl group, and it is particularly preferable for both R¹ and R² to be methyl groups.

X is a halogen atom, an alkyl group having from 1 to 4 carbons, a haloalkyl group having from 1 to 4 carbons, an alkoxy group having from 1 to 4 carbons, a haloalkoxy group having from 1 to 4 carbons, a phenyl group, a cyano group, or a nitro group.

Examples of halogen atoms include chlorine atoms, fluorine atoms, bromine atoms and iodine atoms.

Examples of alkyl groups having from 1 to 4 carbon atoms include methyl groups, ethyl groups, n-propyl groups, 1-methylethyl groups, 2-methylpropyl groups, n-butyl groups and 1,1-dimethylethyl groups.

Examples of haloalkyl groups having from 1 to 4 carbon atoms include trifluoromethyl groups, 1,1,2,2,2-pentafluoroethyl groups, chloromethyl groups, trichloromethyl groups and bromomethyl groups.

Examples of alkoxy groups having from 1 to 4 carbon atoms include methoxy groups, ethoxy groups and n-propoxy groups.

Examples of haloalkoxy groups having from 1 to 4 carbon atoms include trifluoromethoxy groups, difluoromethoxy groups, 1,1,2,2,2-pentafluoroethoxy groups and 2,2,2-trifluoroethoxy groups.

X is preferably a halogen atom, a haloalkyl group having from 1 to 3 carbon atoms, a haloalkoxy group having from 1 to 3 carbon atoms, an alkyl group having from 1 to 3 carbon atoms or an alkoxy group having from 1 to 3 carbon atoms, more preferably a halogen atom, a haloalkyl group having from 1 to 2 carbon atoms or a haloalkoxy group having from 1 to 2 carbon atoms, further preferably a halogen atom, and particularly preferably a fluorine atom or a chlorine atom.

m is an integer from 0 to 5. In cases where m is 2 or higher, the plurality of X groups may be different from each other. Of these, m is preferably an integer from 0 to 3, and more preferably an integer from 0 to 2.

The bonding position of X is not particularly limited, but in cases where m is 1, a bonding position that forms a 4-substituted benzyl group is preferred.

A is a nitrogen atom or a methine group. A is preferably a nitrogen atom.

A preferred example of cycloalkanol derivative (I) is a cycloalkanol derivative represented by general formula (I-1) below, but cycloalkanol derivative (I) is not limited thereto. Moreover, in general formula (I-1), X¹ is a hydrogen atom, a fluorine atom or a chlorine atom.

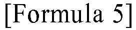

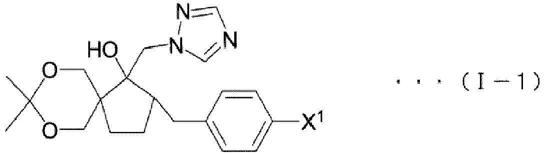

Cycloalkanol derivative (I) can be advantageously used to produce an azole derivative represented by general formula (V) below (hereinafter referred to as azole derivative (V)).

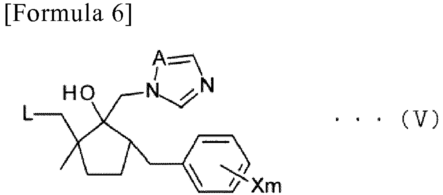

X, m and A in formula (V) above are the same as X, m and A in formula (I). In addition, L is a halogen atom, and a fluorine atom or chlorine atom is preferred as this halogen atom.

Azole derivative (V) exhibits excellent bactericidal activity against many types of bacteria that cause diseases in plants. Moreover, cycloalkanol derivative (I) per se also exhibits excellent bactericidal activity against bacteria that cause diseases in plants. In addition, azole derivative (V) and cycloalkanol derivative (I) can also be advantageously used as agents for protecting industrial materials and as plant growth regulators.

### <Method for producing cycloalkanol derivative (I)>

An explanation will now be given of a method for producing cycloalkanol derivative (I). Here, an explanation will be given of a reaction process for producing cycloalkanol derivative (I) from a cycloalkanone derivative represented by general formula (II) below (hereinafter referred to as cycloalkanone derivative (II)), which is the main characteristic of the present invention. Moreover, R¹, R², X and m in general formula (II) below are the same as in cycloalkanol derivative (I).

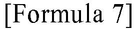

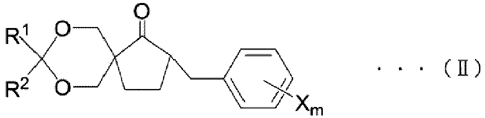

The reaction process for producing cycloalkanol derivative (I) from cycloalkanone derivative (II) includes two reactions shown in Reaction Formula 1 and Reaction Formula 2 below. The reaction shown in Reaction Formula 1 (hereinafter referred to as reaction 1) shows an oxirane production step in which an oxirane derivative represented by general formula (IV) (hereinafter referred to as oxirane derivative (IV)) is produced from cycloalkanone derivative (II). In addition, the reaction shown in Reaction Formula 2 (hereinafter referred to as reaction 2) shows an azolation step in which cycloalkanol derivative (I) is produced from oxirane derivative (IV).

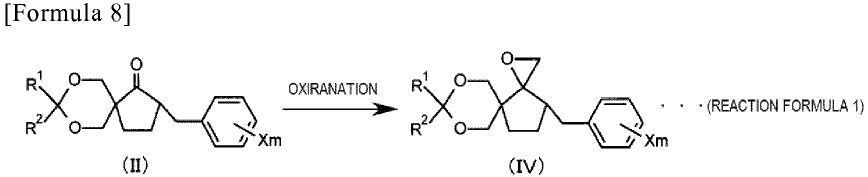

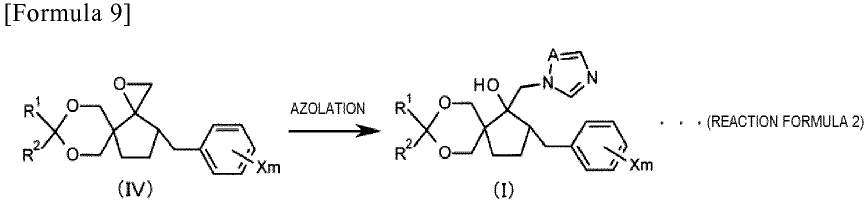

Reaction 1 and reaction 2 may be carried out in the same reaction system or in different reaction systems. Here, "reaction system" means either a single reaction that occurs in a reaction vessel or a plurality of reactions that occur successively in a reaction vessel, and this also applies in the explanations given below. That is, in the method for producing a cycloalkanol derivative of the present embodiment, even in the case of a product obtained from a raw material via an intermediate, reactions that occur successively in a reaction vessel are regarded as being the same reaction system.

More specifically, in cases where reaction 2 is initiated after reaction 1 is complete, reaction 1 and reaction 2 are different reaction systems. In such cases, the vessel in which reaction 1 is carried out and the vessel in which reaction 2 is carried out may be the same or different. Meanwhile, in cases where reaction 2 is initiated while the reaction 1 is ongoing, reaction 1 and reaction 2 occur simultaneously and successively, and the reaction systems are therefore the same. In such cases, the entire quantity of the raw materials for reaction 1 and reaction 2 may be placed in the reaction vessel in advance, or introduced as appropriate during the reactions.

In the present embodiment, reactions that take place in the same reaction system are known as "one-pot (reactions)", and reactions that take place in different reaction systems are known as "stepwise (reactions)".

That is, it can be said that reaction 1 and reaction 2 may be one-pot reactions or stepwise reactions. Here, an explanation will now be given of a case in which reaction 1 and reaction 2 are one-pot reactions.

### (One-pot reaction)

Reaction Formula 3 below shows a reaction formula for an azolylmethylation reaction using a one-pot reaction. In Reaction Formula 3, A is a nitrogen atom or methine group, and M is an alkali metal atom. A sodium atom is preferred as the alkali metal atom.

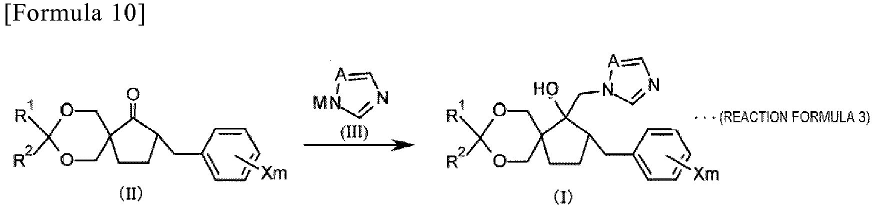

As shown in Reaction Formula 3, cycloalkanol derivative (I) is obtained in a one-pot reaction by azolylmethylating cycloalkanone derivative (II) using azole compound (III) in the presence of a sulfur ylide. Specifically, oxirane derivative (IV) is produced from cycloalkanone derivative (II) by the action of the sulfur ylide in the one-pot reaction. Next, the obtained oxirane derivative (IV) reacts with azole compound (III), which is present in the reaction system, thereby forming cycloalkanol derivative (I).

Next, a detailed explanation will be given of a one-pot reaction. First, cycloalkanone derivative (II) and azole compound (III) are dissolved in a solvent. A sulfonium compound or sulfoxonium compound is added to the obtained solution, and an alkali metal methoxide is then also added to the solution, thereby producing a sulfur ylide in the reaction system. By producing the sulfur ylide, oxirane derivative (IV) is produced from cycloalkanone derivative (II) in the reaction system. The obtained oxirane derivative (IV) reacts with azole compound (III), which is present in the solvent, thereby forming cycloalkanol derivative (I). Specifically, cycloalkanol derivative (I) is produced by a carbon-nitrogen bond being formed between a carbon atom that constitutes the oxirane ring in oxirane derivative (IV) and a nitrogen atom in azole compound (III). Moreover, the alkali metal methoxide may be divided and added in separate portions.

In addition, the sulfonium compound or sulfoxonium compound may, like the alkali metal methoxide, be divided and added in separate portions. Furthermore, it is possible to divide both the alkali metal methoxide and the sulfonium compound or sulfoxonium compound, and add these in separate portions.

In the present embodiment, the sulfur ylide is produced in the reaction system as a result of the alkali metal methoxide reacting with the sulfonium compound or sulfoxonium compound, and the type of sulfur ylide produced depends on the type of sulfonium compound or sulfoxonium compound used.

Examples of the sulfonium compound or sulfoxonium compound include trimethylsulfonium bromide, trimethylsulfonium chloride, trimethylsulfonium iodide, trimethylsulfoxonium bromide, trimethylsulfoxonium chloride and trimethylsulfoxonium iodide. From the perspective of yield when producing the sulfur ylide, the use of a sulfoxonium compound is preferred, and of these, trimethylsulfoxonium bromide is more preferred.

The use of sodium methoxide as the alkali metal methoxide is preferred. In addition, from the perspective of ease of handling during production, it is preferable to use the sodium methoxide in the form of a solution. It is more preferable for this solution to be a methanol solution of sodium methoxide.

Moreover, examples of solvents used in the one-pot reaction in the present embodiment include amide bond-containing polar solvents, dimethylsulfoxide, and mixed solvents of alcohols and these polar solvents. Examples of amide bond-containing polar solvents include N-methylpyrrolidone, N, N-dimethylacetamide and N,N-dimethylformamide. In addition, examples of alcohols include t-butanol. Furthermore, the solvent used in the above-mentioned reaction may be mixed with toluene.

The reaction temperature and reaction time can be set as appropriate according to the type of solvent used, the type of cycloalkanone derivative (II), the type of sulfonium compound or sulfoxonium compound, and the type of alkali metal methoxide. The reaction temperature is preferably from 0 to 200°C, and more preferably from 20 to 100°C. In addition, the reaction time is preferably between 0.1 hours and several days, and more preferably between 0.5 hours and 2 days.

In addition, the number of times the alkali metal methoxide is added is not particularly limited as long as the desired objective can be achieved. The number of times the alkali metal methoxide is added is preferably from 1 to 20, and more preferably from 2 to 10. The same applies in cases where the sulfonium compound or sulfoxonium compound is divided and added in separate portions.

The total usage quantity of the sulfonium compound or sulfoxonium compound is preferably from 0.5 to 5 times, and more preferably from 0.8 to 2 times, the molar quantity of cycloalkanone derivative (II). The usage quantity of azole compound (III) is preferably from 0.5 to 10 times, and more preferably from 0.8 to 5 times, the molar quantity of cycloalkanone derivative (II).

### (Stepwise reaction)

An explanation will now be given of a case in which reaction 1 and reaction 2 are carried out in a stepwise reaction rather than a one-pot reaction.

In cases in which reaction 1 and reaction 2 are carried out in a stepwise reaction, the oxiranation of cycloalkanone derivative (II) (Reaction Formula 1) in the one-pot reaction and the azolation of oxirane derivative (IV) obtained by the oxiranation (Reaction Formula 2) in the one-pot reaction are carried out in different reaction systems. That is, the stepwise reaction differs from the one-pot reaction in that azole compound (III) is not present in the reaction vessel when oxirane derivative (IV) is produced. The reagents, solvents, reaction conditions, and the like used in the stepwise reaction are the same as those used in the one-pot reaction mentioned above, and detailed explanations for these matters are therefore omitted.

Here, azole compound (III) may be produced from a triazole or imidazole in the reaction system. For example, in cases where M is sodium, compound (III) can be produced by reacting sodium hydroxide with a triazole or imidazole. In addition, it is possible to use sodium methoxide instead of sodium hydroxide.

Moreover, it is preferable for cycloalkanol derivative (I) to be produced in a one-pot reaction. When producing cycloalkanol derivative (I) in a one-pot reaction, it is possible to prevent by-products (for example, oxetane derivatives), which are produced in reaction 2 when carrying out a stepwise reaction, from being produced. In this way, it is possible to prevent a reduction in the yield of cycloalkanol derivative (I).

As explained above, by using an alkali metal methoxide, which is less expensive than a conventionally used alkali metal butoxide, in the production method of the present invention, it is possible to produce cycloalkanol derivative (I) with a high yield and with no concerns regarding the production of by-products. In this way, it is possible to produce cycloalkanol derivative (I) (and even azole derivative (V) shown below) at low cost. In addition, by using the alkali metal methoxide in the form of a solution, the alkali metal methoxide can be handled more easily during production than a solid alkali metal methoxide.

### <Scheme for producing cycloalkanol derivative (I)>

An explanation will now be given of a process for producing cycloalkanol derivative (I) other than by the above-mentioned azolylmethylation reaction. Moreover, cycloalkanol derivative (I) is hereinafter described as compound (6). Compound (6) can be produced according to scheme 1 below from compound (1), which is obtained using a publicly known technique (for example, that disclosed in Patent Document 3).

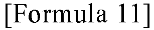

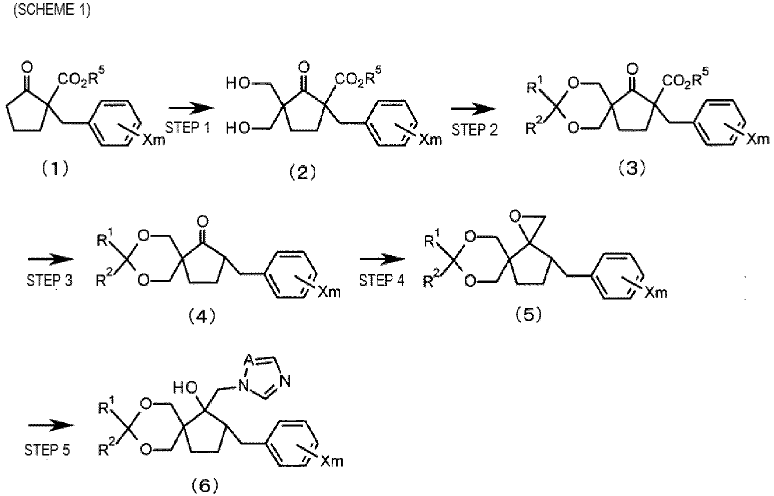

The various steps will now be explained.

### (Step 1: Hydroxymethylation step)

In step 1, a compound represented by general formula (2) (hereinafter referred to as compound (2)) is obtained by subjecting a compound represented by general formula (1) (hereinafter referred to as compound (1)) to hydroxymethylation (see Reaction Formula 4 below). Here, X and m are as described above. R⁵ is an alkyl group having from 1 to 4 carbon atoms.

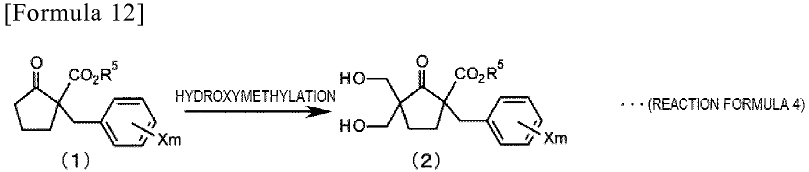

The method for subjecting compound (1) to hydroxymethylation can be a method in which compound (1) is reacted with formaldehyde or a formaldehyde derivative in a solvent in the presence of a base.

The base can be an alkali metal carbonate such as sodium carbonate or potassium carbonate, an alkali metal hydroxide such as sodium hydroxide, or an organic base such as triethylamine, but is not limited to these. The usage quantity of the base is, for example, from 0.01 to 10 times, and preferably from 0.1 to 5 times, the molar quantity of compound (1).

The reaction temperature is, for example, from 0 to 250°C, and preferably from 0 to 100°C. The reaction time is, for example, between 0.1 hours and several days, and preferably between 0.5 hours and 2 days.

The solvent is not particularly limited, but it is possible to use an ether, such as diethyl ether, tetrahydrofuran (THF) or dioxane, an aromatic hydrocarbon, such as benzene, toluene or xylene, an alcohol, such as methanol or ethanol, or water, or the like, and mixtures of these solvents can be used if necessary. Here, if the reaction system forms a dual phase, it is preferable to use a phase transfer catalyst such as, for example, a commonly used quaternary ammonium salt (for example, benzyltriethylammonium chloride).

Examples of formaldehyde derivatives include paraformaldehyde, 1,3,5-trioxane, formaldehyde dialkyl acetal, and the like.

The usage quantity of the formaldehyde or formaldehyde derivative is, for example, from 1 to 40 times, and preferably from 1.6 to 20 times, the molar quantity of compound (1).

### (Step 2: Protecting group introduction step)

In step 2, protecting groups that simultaneously protect the hydroxyl groups in the two hydroxyethyl groups in compound (2) are introduced by means of a single compound, thereby obtaining a compound represented by general formula (3) (hereinafter referred to as compound (3)) (see Reaction Formula 2 below). Here, X, m, R¹, R² and R⁵ are as described above.

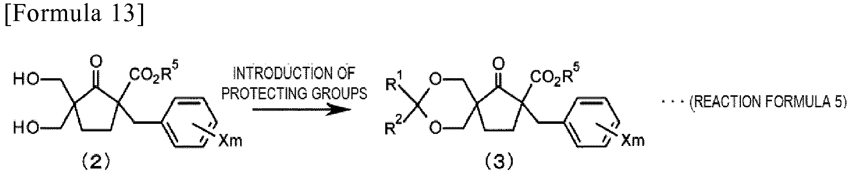

The method for introducing protecting groups to the hydroxyl groups in compound (2) can be a method involving reacting compound (2) with an acetal or ketone in the presence of an acid.

The acetal can be a compound represented by general formula (VI) below.

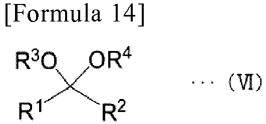

In addition, the ketone can be a compound represented by general formula (VII) below.

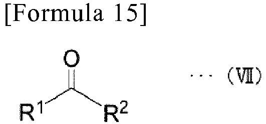

In formula (VI) and formula (VII), R¹ and R² are the same functional groups as those represented by R¹ and R² in the azole derivative being produced. In formula (VI), R³ and R⁴ are each independently an alkyl group having from 1 to 4 carbon atoms, such as a methyl group or an ethyl group.

The usage quantity of the acetal or ketone is, for example from 0.5 to 20 times, and preferably from 0.8 to 10 times, the molar quantity of compound (2).

The acid can be an inorganic acid such as hydrochloric acid, phosphoric acid or sulfuric acid, or an organic acid such as p-toluenesulfonic acid or trifluoroacetic acid. The usage quantity of the acid is, for example, from 0.001 to 10 times, and preferably from 0.002 to 2 times, the molar quantity of compound (2).

The reaction temperature is, for example, from 0 to 200°C, and preferably from 0 to 100°C. The reaction time is, for example, between 0.1 hours and several days, and preferably between 0.5 hours and 2 days.

### (Step 3: Hydrolysis/decarboxylation step)

In step 3, compound (4) is produced by subjecting compound (3) to hydrolysis/decarboxylation (see Reaction Formula 6 below). Moreover, compound (4) below is the same as cycloalkanone derivative (II).

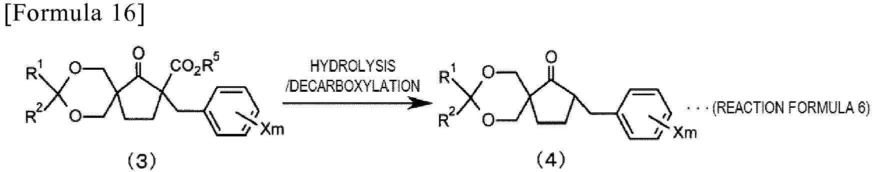

The method for subjecting compound (3) to hydrolysis/decarboxylation can be a method involving reacting compound (3) in a solvent in the presence of a base.

It is preferable to use an alkali metal base, such as sodium hydroxide or potassium hydroxide, as the base. The usage quantity of the base is, for example, from 0.1 to 50 times, and preferably from 0.2 to 20 times, the molar quantity of compound (3).

The solvent can be water, water to which an alcohol is added, or a solvent composition consisting of solvents that do not form a homogeneous layer (water-toluene, or the like). In cases where solvents that do not form a homogeneous layer are used, it is possible to use a phase transfer catalyst (for example, a commonly used quaternary ammonium salt) in the reaction system.

The reaction temperature is, for example, from 0°C to the reflux temperature, and preferably from 20°C to the reflux temperature. The reaction time is, for example, between 0.1 hours and several days, and preferably from 0.5 to 24 hours.

### (Step 4 / Step 5: Azolylmethylation process)

The azolylmethylation process carried out in step 4 and step 5 has already been explained in detail, and an explanation of this process will be omitted here.

### <Method for producing azole derivative (V)>

An explanation will now be given of a method for producing the above-mentioned azole derivative (V). Moreover, azole derivative (V) is hereinafter described as compound (10). It is preferable to use the above-mentioned compound (6) as an intermediate in the production of compound (10).

Compound (10) can be produced from compound (6) according to Scheme 2 described below. Because compound (10) may be produced by using publicly known methods (for example, see Patent Document 1) for step 7 and subsequent steps, only step 6 in the present embodiment will now be explained.

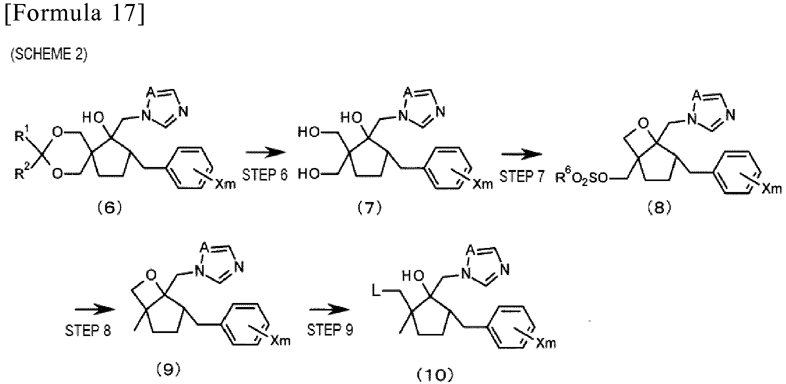

### (Step 6: Deprotection step)

In step 6, compound (7) is produced from compound (6) by deprotecting compound (6) (see Reaction Formula 7 below).

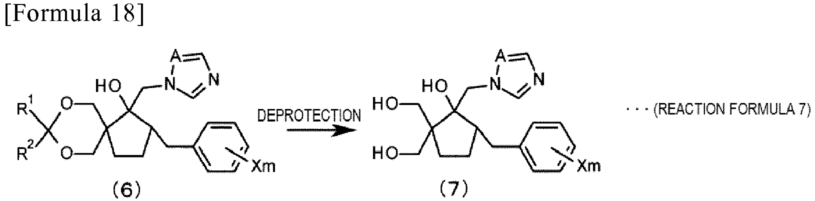

Here, X, m, R¹, R² and A are as described above.

The method for deprotecting the protecting groups on compound (6) can be a method that involves the use of an acid.

The acid is preferably an inorganic acid, for example a hydrogen halide such as hydrogen chloride, sulfuric acid, or the like. The usage quantity of the acid is not particularly limited, but is, for example, from 0.01 to 100 times, and preferably from 0.1 to 20 times, the molar quantity of compound (6).

The reaction temperature is, for example, from 0 to 200°C, and preferably from 20 to 100°C. In addition, the reaction time is, for example, between 0.1 hours and several days, and preferably between 0.5 hours and 2 days.

Therefore, according to the method for producing an azole derivative according to the present invention, as described above, it is possible to obtain compound (6) (cycloalkanol derivative (I)) conveniently, at low cost, and with a high yield. In addition, by using compound (6), it is possible to obtain compound (10) (azole derivative (V)) by using a publicly known method.

### (Additional matters)

The present invention is not limited to the embodiment described above, and may be variously altered within the scope indicated in the claims. That is, embodiments obtained by combining appropriately altered technical means within the scope indicated in the claims are encompassed by the technical scope of the present invention.

### EXAMPLES

The present invention will now be explained in greater detail through the use of production examples. Moreover, the present invention is not limited to the production examples given below, as long as the gist of the invention is not exceeded.

### <Production Example 1: Synthesis of 2-(4-chlorobenzyl)-8,8-dimethyl-7,9-dioxaspiro[4,5]decan-1-one (compound 4-a; R¹ = CH₃, R² = CH₃, Xₘ = 4-Cl in compound (4))>

### [1] Synthesis of 1-(4-chlorobenzyl)-3,3-bis(hydroxymethyl)-2-oxocyclopentane carboxylic acid methyl ester (compound 2-a; R⁵ = CH₃, Xₘ = 4-Cl in compound (2)) [Step 1]

10.38 g of potassium carbonate and 65 mL of a 37% aqueous solution of formalin were added to 79.98 g of 1-(4-chlorobenzyl)-2-oxocyclopentane carboxylic acid methyl ester (compound 1-a; R⁵ = CH₃, Xₘ = 4-Cl in compound (1)), and stirred at room temperature for 23 hours. 250 mL of water and 51 mL of concentrated hydrochloric acid were added to the reaction liquid and stirred for a further 7 hours. Following completion of the reaction, water was added and extraction with ethyl acetate was carried out. The organic layer was washed with water and a saturated saline solution, and then dried with anhydrous sodium sulfate. The solvent was distilled off, thereby obtaining 110.77 g of a crude extract of compound 2-a.

### [2] Synthesis of 2-(4-chlorobenzyl)-8,8-dimethyl-1-oxo-7,9-dioxaspiro[4,5]decane-2-carboxylic acid methyl ester (compound 3-a; R¹ = CH₃, R² = CH₃, R⁵ = CH₃, Xₘ = 4-Cl in compound (3)) [Step 2]

90 mL of acetone dimethyl acetal, 180 mL of toluene and 1.43 g of p-toluenesulfonic acid monohydrate were added to the entire quantity of the crude extract of compound 2-a obtained in [1] above, stirred for 1 hour at 55°C, and then stirred for a further 15 hours at room temperature. Saturated aqueous sodium bicarbonate, water and toluene were added to the reaction liquid, thereby causing partitioning. The aqueous layer was extracted with toluene, and the organic layer was dried with anhydrous sodium sulfate. The solvent was distilled off, thereby obtaining 111.05 g of a crude extract of compound 3-a.

### [3] Synthesis of compound 4-a [Step 3]

8 mL of toluene was added to 48.0 g of the crude extract of compound 3-a obtained in [2] above, and the crude extract was dissolved by being heated to 100°C. Here, 80.0 g of a 25 wt.% aqueous solution of sodium hydroxide was added to the obtained solution, a reaction was carried out under reflux for 2 hours, and the reaction liquid was then allowed to cool. Water was added to the reaction liquid, and extraction with toluene was then carried out. The organic layer was washed with a mixture obtained by mixing 150 mL of water with 150 mL of saturated aqueous ammonium chloride, and then dried with anhydrous sodium sulfate. The solvent was distilled off, thereby obtaining compound 4-a.
Yield: 27.62 g

### <Production Example 2: Synthesis of 2-benzyl-8,8-dimethyl-1-(1H-1,2,4-triazol-1-ylmethyl)-7,9-dioxaspiro[4,5]decan-1-ol (azole derivative 6-a; R¹ = CH₃, R² = CH₃, Xₘ = 4-Cl in compound (6))>[One-pot reaction of Step 4 and Step 5]

Production Example 2-1 (method "A") and Production Example 2-2 (method "B"), in which the production conditions were different, are shown as Production Example 2. In addition, Comparative Production Example 2-1 (method "A") and Comparative Production Example 2-2 (method "B") are shown as Comparative Production Example 2.

Moreover, method "A" is a method in which both the base (sodium methoxide or sodium tert-butoxide) and TMSOB were divided and added in separate portions, and method "B" is a method in which, of the base and the TMSOB, only the base was divided and added in separate portions.

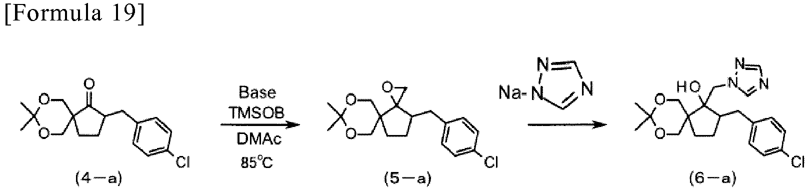

### (1) Production Example 2-1

3.26 mmol of compound 4-a was dissolved in 3.2 mL of dimethylacetamide, and heated to 85°C. 4.91 mmol of a sodium salt of 1, 2, 4-triazole was added to the obtained solution. Next, 1.99 mmol of a 28% methanol solution of sodium methoxide and 4.78 mmol of trimethylsulfoxonium bromide (TMSOB) were added intermittently over a period of 2 hours, after which a reaction was allowed to progress for 1 hour. Following completion of the reaction, water was added to the reaction liquid, and extraction with ethyl acetate was then carried out. The organic layer was washed with water and a saturated saline solution, and then dried with anhydrous sodium sulfate. The solvent was distilled off, and the crude product was purified by means of silica gel chromatography, thereby obtaining azole derivative 6-a as a mixture of isomers (cis isomer : trans isomer ratio = 93 : 7). The purity was 95.9%.

Here, the "cis isomer" and "trans isomer" occur as a result of the steric configuration of the hydroxyl group and benzyl group bonded to the cyclopentane ring in azole derivative (6). In addition, the "cis isomer" and "trans isomer" in subsequent production examples occur as a result of the steric configuration of a group corresponding to this hydroxyl group (or oxetane group) and a group corresponding to this benzyl group in the compounds in question.

### (2) Production Example 2-2

3.24 mmol of compound 4-a was dissolved in 3.2 mL of dimethylacetamide, and heated to 85°C. 4.85 mmol of a sodium salt of 1,2,4-triazole and 4.53 mmol of TMSOB were added to the obtained solution. Next, 1.64 mmol of a 28% methanol solution of sodium methoxide was added intermittently over a period of 50 minutes, after which a reaction was allowed to progress for 50 minutes. Following completion of the reaction, azole derivative 6-a was obtained using the same method as that used in Production Example 2-1 above. The purity was 99.1%.

### (3) Comparative Production Example 2-1

3.24 mmol of compound 4-a was dissolved in 3.2 mL of dimethylacetamide, and heated to 85°C. 4.85 mmol of a sodium salt of 1,2,4-triazole was added to the obtained solution. Next, 1.94 mmol of sodium tert-butoxide and 4.78 mmol of TMSOB were added intermittently over a period of 2 hours, after which a reaction was allowed to progress for 1 hour. Following completion of the reaction, azole derivative 6-a was obtained using the same method as that used in Production Example 2-1 above. The purity was 98.7%.

### (4) Comparative Production Example 2-2

3.24 mmol of compound 4-a was dissolved in 3.2 mL of dimethylacetamide, and heated to 85°C. 4.88 mmol of a sodium salt of 1,2,4-triazole and 4.54 mmol of TMSOB were added to the obtained solution. Next, 1.63 mmol of sodium tert-butoxide was added intermittently over a period of 50 minutes, after which a reaction was allowed to progress for 50 minutes. Following completion of the reaction, azole derivative 6-a was obtained using the same method as that used in Production Example 2-1 above. The purity was 98.3%.

Table 1 shows the yields of azole derivative 6-a produced in the production examples.

**[Table 1]**

| | Base | | Method | Yield[%] |
|---|---|---|---|---|
| Production Example 2-1 | MeONa | Liquid | A | 75.5 |
| Production Example 2-2 | MeONa | Liquid | B | 78.2 |
| Comparative Production Example 2-1 | t-BuONa | Solid | A | 73.4 |
| Comparative Production Example 2-2 | t-BuONa | Solid | B | 77.4 |

### <Production Example 3: Synthesis of 5-(4-chlorobenzyl)-2,2-bis(hydroxymethyl)-1-(1H-1,2,4-triazol-1-ylmethyl)cyclopentanol (azole derivative 7-a; Xₘ = 4-Cl, A = N in compound (7))>[Step 6]

8.98 g of azole derivative 6-a was dissolved in a mixture of 30 mL of methanol and 40 mL of a 6N aqueous solution of hydrochloric acid, and stirred at room temperature for 4 hours. Water was added to the reaction liquid, after which the reaction liquid was neutralized with sodium carbonate and sodium bicarbonate. After this was extracted with ethyl acetate, the organic layer was washed with saturated saline. The reaction liquid was then dried with anhydrous sodium sulfate, after which the solvent was distilled off, thereby obtaining azole derivative 7-a as a mixture of isomers. The yield was 7.96 g.

## Claims

1. method for producing a cycloalkanol derivative, by which a cycloalkanol derivative represented by general formula (I) below is produced, the method comprising an azolylmethylation reaction step of azolylmethylating a cycloalkanone derivative represented by general formula (II) below using an azole compound represented by general formula (III) below in the presence of a sulfur ylide, and
the sulfur ylide being produced from a sulfonium compound or sulfoxonium compound and an alkali metal methoxide in a reaction system in the azolylmethylation reaction step 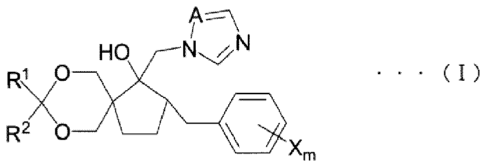 (In formula (I) above, R¹ and R² are each independently a hydrogen atom, an alkyl group having from 1 to 4 carbon atoms, a phenyl group or a benzyl group, and one or more hydrogen atoms on these phenyl groups and benzyl groups may be substituted with alkyl groups having from 1 to 4 carbon atoms, alkoxy groups having from 1 to 4 carbon atoms or halogen atoms. In addition, A is a nitrogen atom or a methine group. X is a halogen atom, an alkyl group having from 1 to 4 carbon atoms, a haloalkyl group having from 1 to 4 carbon atoms, an alkoxy group having from 1 to 4 carbon atoms, a haloalkoxy group having from 1 to 4 carbon atoms, a phenyl group, a cyano group or a nitro group, and m is an integer from 0 to 5. In cases where m is 2 or higher, the plurality of X groups may be different from each other) 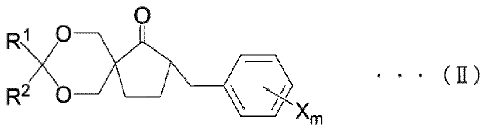 (In formula (II) above, R¹, R², X and m are the same as R¹, R², X and m in formula (I) above) 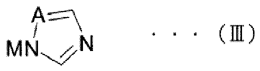 (In formula (III) above, M is an alkali metal atom and A is the same as A in formula (I) above).

2. The method for producing a cycloalkanol derivative according to claim 1, the azolylmethylation reaction step including
an oxirane production step of oxiranating the cycloalkanone derivative in the presence of a sulfur ylide so as to produce an oxirane derivative represented by general formula (IV) below, and an azolation step of reacting the oxirane derivative produced in the oxirane production step with the azole compound, and
the oxirane production step and the azolation step being carried out in the same reaction system 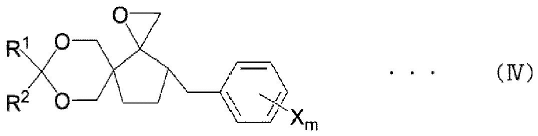 (In formula (IV) above, R¹, R², X and m are the same as R¹, R², X and m in formula (I) above).

3. The method for producing a cycloalkanol derivative according to claim 1, the azolylmethylation reaction step including
an oxirane production step of oxiranating the cycloalkanone derivative in the presence of a sulfur ylide so as to produce an oxirane derivative represented by general formula (IV) below, and
an azolation step of reacting the oxirane derivative produced in the oxirane production step with the azole compound, and
the oxirane production step and the azolation step being carried out in different reaction systems 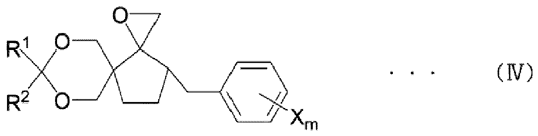 (In formula (IV) above, R¹, R², X and m are the same as R¹, R², X and m in formula (I) above).

4. The method for producing a cycloalkanol derivative according to any one of claims 1 to 3, wherein the alkali metal methoxide is in the form of a solution.

5. The method for producing a cycloalkanol derivative according to any one of claims 1 to 4, wherein the alkali metal methoxide is divided and added to the reaction system in separate portions.

6. The method for producing a cycloalkanol derivative according to any one of claims 1 to 5, wherein the sulfonium compound or sulfoxonium compound is divided and added to the reaction system in separate portions.

7. The method for producing a cycloalkanol derivative according to any one of claims 1 to 6, wherein the alkali metal methoxide is sodium methoxide.

8. The method for producing a cycloalkanol derivative according to any one of claims 1 to 7, wherein at least one compound selected from among trimethylsulfoxonium chloride, trimethylsulfoxonium bromide and trimethylsulfoxonium iodide is used as the sulfoxonium compound.

9. A method for producing an azole derivative represented by general formula (V) below, the method including the method for producing a cycloalkanol derivative according to any one of claims 1 to 8 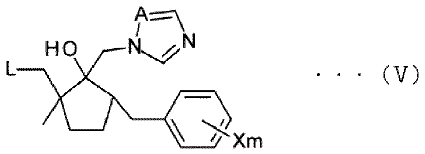 (X, m and A in formula (V) above are the same as X, m and A in formula (I) above and in addition, L is a halogen atom).
